**Europäisches Patentamt**

**European Patent Office**

**Office européen des brevets**

(11) Numéro de publication : **0 519 836 A1**

# (12) DEMANDE DE BREVET EUROPEEN

(21) Numéro de dépôt : **92401729.6**

(22) Date de dépôt : **19.06.92**

(51) Int. Cl.⁵ : **A61B 5/103**

(30) Priorité : **20.06.91 FR 9107588**

(43) Date de publication de la demande :
**23.12.92 Bulletin 92/52**

(84) Etats contractants désignés :
**BE DE GB IT NL**

(71) Demandeur : **ATCOM Sarl**
**80, rue des Bas Avaux**
**F-45800 Saint-Jean de Braye (FR)**

(72) Inventeur : **Junca, Claude**
**24 Boulevard Jean Mermoz**
**F-45800 Saint-Jean de Braye (FR)**
Inventeur : **Mansion, Marcel**
**109 Rue des Gatinettes**
**F-45590 Saint-Cyr-en-Val (FR)**

(74) Mandataire : **Bruder, Michel et al**
**Cabinet Michel Bruder Conseil en Brevets 10,**
**rue de la Pépinière**
**F-75008 Paris (FR)**

(54) **Appareil de détection et de correction des anomalies d'équilibre d'un patient.**

(57) La présente invention concerne un appareil de détection et de correction des anomalies d'équilibre d'un patient, du type comportant un premier plateau inférieur (1), fixe par rapport au sol, un second plateau mobile (5) maintenu par rapport au premier par des moyens déformables élastiques (7), sur lequel prend place un patient, des premiers moyens de visualisation (14) de l'horizontalité du plateau mobile (5), du type niveau à bulle, et des seconds moyens de visualisation.

Cet appareil est caractérisé en ce que les seconds moyens de visualisation sont constitués d'un inclinomètre (13), lié mécaniquement aux premiers moyens de visualisation (14) par des moyens susceptibles de ne subir aucune déformation lorsque le patient est en position sur le plateau mobile (5), l'inclinomètre (13) étant apte à fournir des signaux dits en (X) et en (Y) relatifs au positionnement du plateau mobile (5) par rapport à l'horizontale, suivant aux moins deux axes orthogonaux (X,Y), ces signaux étant traités par des moyens électroniques, aptes à fournir une indication sur la position du plateau mobile (5) par rapport à l'horizontale suivant ces deux axes orthogonaux.

FIG. 1

EP 0 519 836 A1

Jouve, 18, rue Saint-Denis, 75001 PARIS

La présente invention concerne un appareil destiné à détecter les anomalies d'équilibre d'un patient, en vue de les traiter et de les corriger.

On connaît de tels appareils, ou planches d'équilibre, qui sont habituellement constitués de deux plateaux, à savoir un premier plateau disposé de façon fixe sur le sol, et un second plateau mobile, maintenu sur celui-ci par l'intermédiaire d'une structure déformable, telle que des ressorts ou des barres de torsion, sur lequel prend place le patient. Le plateau mobile est habituellement pourvu d'un système d'affichage de niveau, de type niveau à bulle, permettant de réaliser, en début de séance, son positionnement de façon rigoureusement horizontale. Des moyens permettant de détecter tout mouvement du plateau mobile par rapport au plateau fixe sont prévus. Ces moyens sont habituellement constitués de capteurs de déplacement ou de proximité, de type inductif, optique, ou capacitif. Afin d'être en mesure de détecter l'inclinaison du plateau mobile suivant deux axes, ces dispositifs mettent en oeuvre au moins deux capteurs, ce qui conduit en raison des défauts de linéarité inhérents à chacun d'eux, à une dégradation de la mesure fournie, ce qui impose un équilibrage manuel des zéros des capteurs lors de l'opération de réglage de mise à niveau.

Une autre source d'erreur des planches d'équilibre de l'art antérieur, provient de ce que, sous l'effet du poids du patient, celles-ci se déforment si bien qu'elles engendrent, de ce fait, une variation entre l'indication donnée par le niveau à bulle et celle fournie par les moyens capteurs de déplacement. Cette déformation n'étant pas constante, puisqu'elle varie en fonction du positionnement du patient sur la planche mobile, il s'ensuit que la correction à fournir pour obtenir une concordance entre les deux moyens indicateurs ne peut être réglée de façon efficace.

Une solution consiste à renforcer la structure du plateau mobile sur lequel prend place le patient, mais cette solution conduit à une augmentation, à la fois du poids, de l'encombrement et du prix de revient de l'appareil.

La présente invention a pour but de proposer un appareil permettant de détecter les anomalies de positionnement du corps d'un patient, permettant d'éviter les erreurs dues au décalage entre les indications fournies par le niveau à bulle et les moyens capteurs de déplacement, tout en permettant de réaliser le plateau mobile avec des matériaux lui conférant une épaisseur inférieure à celle de la technique antérieure.

La présente invention a ainsi pour objet un appareil de détection et de correction des anomalies d'équilibre d'un patient, du type comportant un premier plateau inférieur, fixe par rapport au sol, un second plateau mobile, maintenu par rapport au premier par des moyens déformables élastiques, sur lequel prend place un patient, des premiers moyens de visualisation de l'horizontalité du plateau mobile, du type niveau à bulle, et des seconds moyens de visualisation, caractérisé en ce que les seconds moyens de visualisation sont constitués d'un inclinomètre, lié mécaniquement aux premiers moyens de visualisation par des moyens susceptibles de ne subir aucune déformation lorsque le patient est en position sur le plateau mobile, l'inclinomètre étant apte à fournir des signaux dits en X et en Y relatifs au positionnement du plateau mobile par rapport à l'horizontale, suivant au moins deux axes orthogonaux, ces signaux étant traités par des moyens électroniques, aptes à fournir une indication sur la position du plateau mobile par rapport à l'horizontale suivant ces deux axes orthogonaux.

Dans une variante intéressante de l'invention les moyens de contrôle de positionnement sont constitués d'un écran d'affichage, de préférence à cristaux liquide, générant un spot de contrôle au centre de son écran lorsque le plateau mobile se situe dans une position parfaitement mobile, par rapport à une position de référence.

Dans une autre variante de l'invention l'appareil comporte des moyens de temporisation assurant la coupure de son alimentation électrique lorsque les signaux en X et les signaux en Y restent constants pendant un temps prédéterminé.

On décrira ci-après, à titre d'exemple non limitatif, une forme d'exécution de la présente invention, en référence au dessin annexé sur lequel :

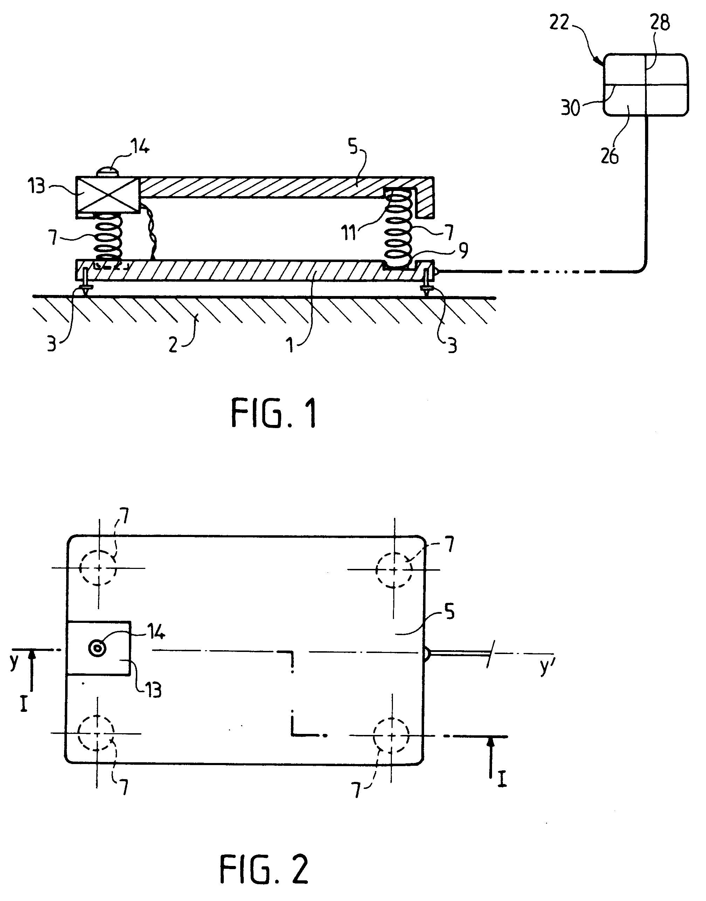

La figure 1 est une vue en coupe verticale brisée, suivant la ligne I,I de la figure 2, d'un appareil suivant l'invention.

La figure 2 est une vue du dessus de l'appareil suivant l'invention.

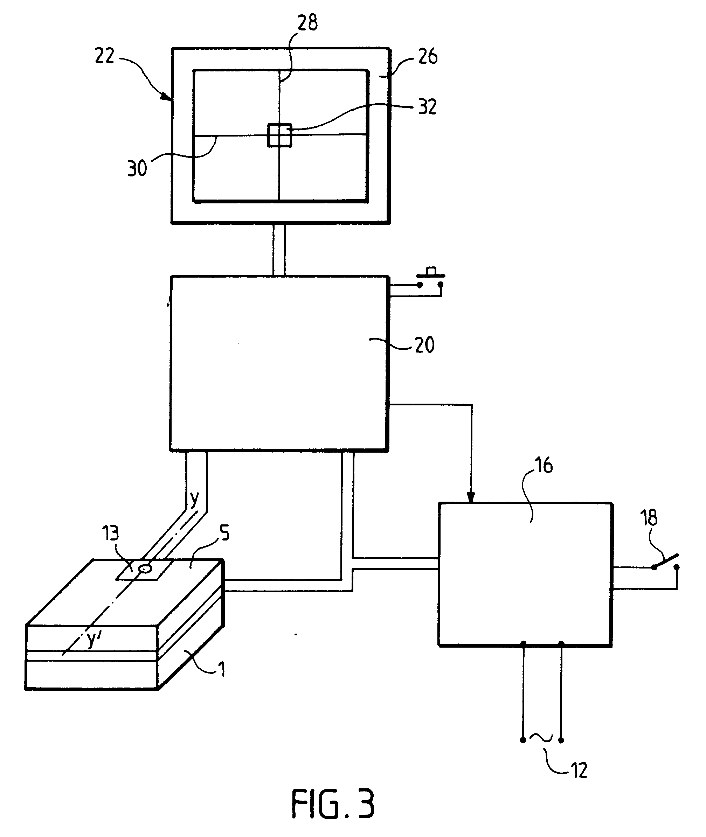

La figure 3 est une vue schématique de l'appareil suivant l'invention.

Le dispositif suivant l'invention est constitué d'un premier plateau 1, ou plateau de base, en appui sur le sol 2 par quatre pieds 3, réglables en hauteur, sur lequel prend appui un second plateau 5, ou plateau mobile, par l'intermédiaire de quatre ressorts de compression 7 disposés à ses quatres coins.

Les extrémités des ressorts de compression 7 prennent place dans des cavités 9,11, respectivement prévues sur les faces internes des premier et second plateaux 1 et 5, dans lesquelles ils sont fixés, par des moyens non représentés sur le dessin. La raideur des ressort de compression 7 est déterminée de façon telle que l'utilisateur ne puisse amener en contact le plateau mobile 5 avec le plateau de base 1.

Le plateau mobile 5 reçoit un inclinomètre 13, constitué d'un boîtier regroupant des moyens électroniques connus en eux-mêmes. Un niveau à bulle 14, de type sphérique, est disposé sur la face supérieure du boîtier de l'inclinomètre 13, de façon qu'il soit visible par le patient lorsque ce dernier prend place sur le plateau mobile 5. On notera que le niveau à bulle 14, est disposé de façon telle qu'aucun élément susceptible de déformation, sous l'action du poids du patient, n'est interposé entre celui-ci et l'inclinomètre 13.

L'alimentation électrique de l'appareil suivant l'invention peut être assurée, soit par une batterie interne, soit par une alimentation externe de type stabilisé, reliée au réseau électrique. Dans le présent mode de mise en oeuvre de l'invention, l'inclinomètre 13 est alimenté par le courant électrique du réseau 12, par l'intermédiaire d'une alimentation stabilisée 16, commandée par un interrupteur 18. Cette alimentation 16 assure également la fourniture de la puissance électrique à des moyens électroniques de traitement 20 des signaux, comprenant un processeur. L'inclinomètre 13 comporte lui-même des moyens électroniques fournissant, en sortie, deux signaux respectifs dits "en X" et "en Y", c'est à dire des signaux qui sont caractéristiques de l'inclinaison de l'inclinomètre 13 par rapport à l'horizontale, respectivement dans le sens transversal et dans le sens longitudinal.

Ces signaux sont numérisés, et sont traités numériquement par le processeur, de façon à déterminer une valeur qui est proportionnelle à l'écart de l'inclinomètre avec l'horizontale, respectivement dans le sens transversal et dans le sens longitudinal. Les moyens électroniques de traitement 20 fournissent également des valeurs égales à la dérivée par rapport au temps de ces signaux.

Les moyens électroniques de traitement 20 sont réunis à des moyens d'affichage 22 constitués, par exemple, d'un boîtier sur la face avant duquel est disposé un écran 26, par exemple à cristaux liquides, cet écran 26 étant constitué d'une multitude de points adressables, ou pixels, par des moyens électroniques de tout type connu.

L'écran d'affichage 26 est traversé, en son centre, par deux lignes repères, respectivement verticale 28 et horizontale 30. La position du plateau principal 5 rapport à l'horizontale, dans le sens longitudinal et dans le sens transversal, est matérialisée sur l'écran 26 par un spot 32 de forme, par exemple, rectangulaire. Ainsi, tout déplacement du spot 32 vers la droite indiquera une inclinaison du plateau mobile 5 vers la droite du patient, et tout déplacement vers le haut du spot 32 indiquera une inclinaison du plateau mobile 5 vers l'avant.

Bien entendu ces déplacements peuvent se combiner, et un déplacement du spot 32 en haut et à droite de l'écran 26 indiquera une inclinaison du plateau mobile 5 à la fois vers l'avant et vers la droite du patient.

Dans ces conditions, la mise en oeuvre du dispositif suivant l'invention se fait ainsi que décrit ci-après.

Après avoir réglé en hauteur les pieds 3 de l'appareil, de façon que le niveau à bulle sphérique 14 indique une parfaite horizontalité de l'inclinomètre 13, on assure la mise à zéro électronique de l'appareil, soit à l'allumage de celui-ci, soit en appuyant sur un bouton d'initialisation 33, ce qui a pour effet de ramener le spot 32 au centre de l'écran 26, c'est-à-dire à l'intersection des lignes 28 et 30. L'appareil est désormais prêt à être mis en oeuvre.

Pour ce faire le patient dispose ses deux pieds sur le plateau mobile 5, de façon que leur axe longitudinal soit orienté dans le sens yy' de l'axe longitudinal du plateau 5. Ainsi, lorsque le patient est parfaitement en équilibre sur le plateau mobile 5, et que le centre de gravité de son corps passe par le centre de ce dernier, le plateau mobile 5 est parfaitement horizontal et le spot 32 est, dès lors, situé au centre de l'écran 26, de même que la bulle du niveau à bulle sphérique 14 se trouve parfaitement centrée par rapport à celui-ci.

Lorsqu'un déséquilibre est mis en évidence par un déplacement du spot 32 sur l'écran de contrôle 26, le patient fait travailler ses muscles de façon à déplacer le centre de gravité de son corps pour ramener le spot 32 au centre de l'écran 26. La répétition de cet exercice permet ainsi au patient de faire travailler certains muscles déterminés de façon par exemple à les fortifier.

L'utilisation d'un inclinomètre en tant que capteur de positionnement du plateau mobile 5 permet de s'affranchir des couplages délicats obtenus jusqu'à présent avec les capteurs de type inductif ou autres. Elle permet de s'affranchir également des phénomènes d'usure qui se manifestent habituellement de façon différente sur les différents capteurs et qui faussent le résultat de la mesure.

Bien entendu, on pourrait disposer le niveau à bulle 14 en un autre endroit du plateau mobile 5, dans la mesure ou l'on choisit cet endroit de façon telle qu'aucune déformation ne soit susceptible de se produire entre celui-ci et l'inclinomètre 13, sous l'effet du poids du patient, de façon à maintenir en parfaite cohérence les indications données par le niveau à bulle 14 et celles fournies par l'indicateur de positionnement.

## Revendications

1.- Appareil de détection et de correction des anomalies d'équilibre d'un patient, du type comportant un premier plateau inférieur (1), fixe par rapport au sol, un second plateau mobile (5), maintenu par rapport au premier par des moyens déformables élastiques (7), sur lequel prend place un patient, des premiers moyens de visualisation (14) de l'horizontalité du plateau mobile (5), du type niveau à bulle, et des seconds moyens de visualisation, caractérisé en ce que les seconds moyens de visualisation sont constitués d'un inclinomètre (13), lié mécaniquement aux premiers moyens de visualisation (14) par des moyens susceptibles de ne subir aucune déformation lorsque le patient est en position sur le plateau mobile (5), l'inclinomètre (13) étant apte à fournir des signaux dits en (X) et en (Y) relatifs au positionnement du plateau mobile (5) par rapport à l'horizontale, suivant aux moins deux axes orthogonaux (X,Y), ces signaux étant traités par des moyens électroniques (20), aptes à fournir une indication sur la position du plateau mobile (5) par rapport à l'horizontale suivant ces deux axes orthogonaux (X,Y).

2.- Appareil suivant la revendication 1 caractérisé en ce que il comporte des moyens aptes, à partir des signaux en (X) et en (Y) fournis par l'inclinomètre (13), à figurer sur un écran d'affichage toute inclinaison du plateau mobile (5) par rapport à l'horizontale suivant les axes (X) et (Y).

3.- Appareil suivant la revendication 1 caractérisé en ce que les premiers moyens de visualisation (14) sont disposés en contact direct avec l'inclinomètre (13).

4.- Appareil suivant la revendication 1 caractérisé en ce que les moyens de traitement électronique (20) des signaux en (X) et en (Y) fournis par l'inclinomètre (13) comportent un processeur apte à former sur les moyens d'affichage (26) un spot (32), à positionner celui-ci au centre de l'écran (26) lorsque le plateau mobile 5 est parfaitement horizontal, à déplacer le spot (32) dans une direction donnée lorsque le plateau (5) bascule vers l'avant, et à le déplacer suivant une seconde direction, perpendiculaire à la première, lorsque le plateau mobile (5) bascule transversalement.

5.- Appareil suivant l'une quelconque des revendications précédentes caractérisé en ce que il comporte des moyens de temporisation assurant la coupure de son alimentation électrique lorsque les signaux en (X) et les signaux en (Y) restent constants pendant un temps prédéterminé.

FIG. 1

FIG. 2

FIG. 3

**Office européen des brevets**

## RAPPORT DE RECHERCHE EUROPEENNE

Numero de la demande

EP 92 40 1729

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | FR-A-2 647 331 (WALTHERT)<br>* page 2, ligne 31 - page 5, ligne 31 *<br>* figures * | 1 | A61B5/103 |
| A | | 2 | |
| A | EP-A-0 119 660 (SNIJDER)<br>* page 7, ligne 24 - page 8, ligne 20 *<br>* page 12, ligne 35 - page 13, ligne 7 *<br>* figures 1,11 * | 1,2,4 | |
| A | FR-A-2 472 929 (KURTZ ET AL.)<br>* page 2, ligne 31 - page 6, ligne 10 *<br>* figures * | 1 | |

**DOMAINES TECHNIQUES RECHERCHES (Int. Cl.5)**

A61B

Le présent rapport a été établi pour toutes les revendications

| Lieu de la recherche | Date d'achèvement de la recherche | Examinateur |
|---|---|---|
| LA HAYE | 28 AOUT 1992 | CHEN A.H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant